(19) Europäisches Patentamt
European Patent Office
Office européen des brevets

(11) Publication number: 0 524 634 B1

# (12) EUROPEAN PATENT SPECIFICATION

(45) Date of publication of patent specification: **19.04.95**

(51) Int. Cl.⁶: **C07C 303/02**, C07C 309/88

(21) Application number: **92112646.2**

(22) Date of filing: **24.07.92**

## (54) Process for the preparation of 1,2-naphtho-quinonediazido-5-sulfonyl chloride.

(30) Priority: **25.07.91 JP 184959/91**

(43) Date of publication of application:
**27.01.93 Bulletin 93/04**

(45) Publication of the grant of the patent:
**19.04.95 Bulletin 95/16**

(84) Designated Contracting States:
**BE DE FR GB IT NL**

(56) References cited:
**EP-A- 0 040 315**
**EP-A- 0 178 356**
**GB-A- 401 643**

**CHEMICAL ABSTRACTS, vol. 96, no. 5, 1 February 1982, Columbus, Ohio, US; abstract no. 34766b, B.Z. YAKOVLEV et al, "Preparation of o-naphthoquinonediazide sulfonyl chlorides", page 621, column 2**

**SYNTHESIS. no. 12, December 1974, Stuttgart, DE, pages 877 - 878; A. BARCO et al, "A New Preparation of Sulfonyl Chlorides via Pyridinium Sulfonates"**

(73) Proprietor: **SUMITOMO CHEMICAL COMPANY, LIMITED**
**5-33, Kitahama 4-chome**
**Chuo-ku**
**Osaka (JP)**

(72) Inventor: **Kamoda, Masaru**
**13-53, Sakaicho-3-chome**
**Niihama-shi (JP)**
Inventor: **Ohishi, Toshiro**
**7-21, Hoshigoecho**
**Niihama-shi (JP)**
Inventor: **Kamemoto, Koichi**
**10-63, Matsuzonocho-3-chome**
**Misawa-shi (JP)**
Inventor: **Haga, Toru**
**3-736, Ikkucho-2-chome**
**Niihama-shi (JP)**

(74) Representative: **VOSSIUS & PARTNER**
**Postfach 86 07 67**
**D-81634 München (DE)**

EP 0 524 634 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing 1,2-naphthoquinonediazidosulfonyl chloride (hereinafter referred to as quinonediazidosulfonyl chloride).

It has been well known long since that quinonediazidosulfonyl chloride is a compound useful as an intermediate of organic industrial chemicals such as chemicals for photography and printing, dyes, liquid crystal, etc. Particularly, with the development of photographic, printing and electronic industries in recent years, the importance of this compound for its use as an intermediate of photosensitizers by taking advantage of its photoreactability is mounting.

The following methods are known for preparing quinonediazidosulfonyl chloride from quinonediazidosulfonic acid:

(1) Quinonediazidosulfonic acid is reacted with a large excess of chlorosulfonic acid.

(2) Quinonediazidosulfonic acid is reacted with chlorine in the presence of chlorosulfonic acid or sulfuric acid anhydride (German Patent Nos. 246,573 and 246,574).

(3) Quinonediazidosulfonic acid is reacted with phosgene in the presence of N,N-dimethylformamide (Japanese Patent Application Kokai No. 59-196860).

Regarding preparations of said quinonediazidosulfonyl chloride involving a reaction of an acid and phosgene, the following methods are known:

An aromatic sulfonic acid is reacted with phosgene in the presence of N,N-dimethylformamide and a tertiary amine such as pyridine, etc. (U.S. Patent No. 3,795,705) (this method being hereinafter referred to as method (4));

A carboxylic acid having 10 or more carbon atoms is reacted with phosgene in the presence of a tertiary amine at a temperature higher than 100 °C (U.K. Patent No. 540,096) (this method being hereinafter referred to as method (5)).

According to said conventional method (1), since there exists an equilibrium between quinonediazidosulfonic acid and quinonediazidosulfonyl chloride produced by the reaction, a large excess of chlorosulfonic acid is required for increasing the forming rate of quinonediazidosulfonyl chloride. According to said conventional method (2), a large excess of chlorine is required. In these methods (1) and (2), the yields of said sulfonyl chloride are about 80%. Further, these methods have the defect that a complicated operation and a long time are required for removal of the unreacted material, so that these methods cannot be necessarily advantageous for an industrial application.

The method (3), in which quinonediazidosulfonic acid is reacted with phosgene in the presence of N,N-dimethylformamide, has the problem that the yield is not satisfactorily high (92%) and that recovery of N,N-dimethylformamide is difficult. The method (4) also has the problem of low yield (90%) and the defect that use of N,N-dimethylformamide is essential and hence its recovery is difficult. In the method (5), although no N,N-dimethylformamide is used, a high temperature of over 100 °C is required for the reaction. Since quinonediazide group is decomposed at such a high temperature, this method can not be applied to the preparation of naphthoquinonediazidosulfonyl chloride.

Accordingly, one object of this invention is to provide a process for preparing quinonediazidosulfonyl chloride in a high yield with a very simple operation.

Another object of this invention is to provide a process for preparing quinonediazidosulfonyl chloride from quinonediazidosulfonic acid or a salt thereof and phosgene without using N,N-dimethylformamide.

These objects could be achieved on the basis of the finding that by carrying out the reaction of quinonediazidosulfonic acid or a salt thereof and phosgene in the presence of pyridine, especially by specifying the amount of pyridine based on quinonediazidosulfonic acid or a salt thereof, it is possible to produce quinonediazidosulfonyl chloride in a high yield with a very simple operation without using N,N-dimethylformamide.

According to the present invention, there is provided a process for preparing 1,2-naphthoquinonediazido-5-sulfonyl chloride represented by the formula (2):

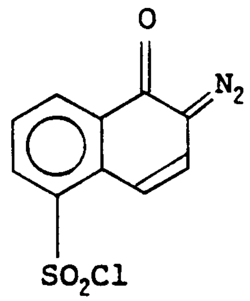

(2)

which comprises reacting 1,2-naphthoquinonediazido-5-sulfonic acid or a salt thereof represented by the formula (1):

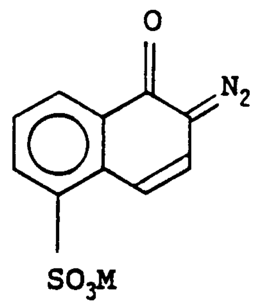

(1)

wherein M represents a hydrogen atom, a sodium or a potassium with phosgene in the presence of pyridine.

In the process according to the present invention, the starting sulfonic acid of formula (1) or a salt thereof is reacted with phosgene to form sulfonyl chloride. The phosgene used in the process of this invention may be either pure phosgene or industrial phosgene. In the above sulfonyl chloride forming reaction, the amount of phosgene used in its reaction with the starting sulfonic acid of formula (1) is about 0.9-1.2 mole, preferably 0.95-1.15 mole, more preferably 1.0-1.1 mole per mole of sulfonic group in the compound of formula (1). When the amount of phosgene used is less than about 0.9 mole per mole of sulfonic group in the compound of formula (1), the reaction is not completed, leaving unreacted compounds of formula (1). When the amount of phosgene exceeds about 1.2 mole, there takes place a side reaction associated with the quinonediazo group, resulting in a reduction of yield.

According to the above method (4), the yield may be low because the ratio of tertiary amine to sulfonic group (tertiary amine/-$SO_3H$ (molar ratio) = 0.068) is small. In the process of this invention, in view of the yield, pyridine is used in a ratio of about 0.3-1 mole, preferably 0.35-0.5 mole, per mole of sulfonic group. When the amount of pyridine is less than about 0.3 mole per mole of sulfonic group, the reaction yield is lowered. When the amount of pyridine is more than about 1 mole per mole of sulfonic group, the reaction yield is not further improved.

The reaction is preferably carried out in a solvent. As the solvent, there is used a lower halogenated hydrocarbon such as methylene chloride, chloroform, carbon tetrachloride, 1,2-dichloroethane or perchloroethylene in view of solubility and yield of the produced sulfonyl chloride and 1,2-dichloroethane is used preferably.

Reaction temperature is not critical, but it is preferably in the range of about 0 to 25 °C, more preferably 5 to 20 °C, most preferably 10 to 15 °C. A lower reaction temperature than the above-defined range results in a low solubility of the produced sulfonyl chloride, then reduces the productivity per unit volume of reactor. The yield is also low when the reaction temperature is higher than 25 °C.

Reaction pressure is also not critical in the process of this invention; the reaction can be carried out either under pressure or under reduced pressure, but it is preferably conducted under around normal pressure. In case the sulfonic acid of formula (1) or a salt thereof used in the sulfonyl chloride-forming reaction contains water, it may be beforehand dehydrated by using phosgene in the presence of the catalyst pyridine used in formation of sulfonyl chloride, and it is desirable to do so.

After the end of the reaction, the reaction mixture is washed with a dilute acid to get rid of pyridine and sodium chloride which are by-products of the reaction. Then the solvent is removed by distillation under reduced pressure and the reaction product is concentrated and crystallized. In this way, the objective 1,2-naphthoquinonediazido-5-sulfonyl chloride with a purity of about 99% can be easily obtained. The pyridine remaining in the dilute acid used for washing can be easily recovered by making said acid alkaline and then distilling it.

According to the process of the present invention, it is possible to easily produce high-purity 1,2-naphthoquinonediazido-5-sulfonyl chloride in a high yield with no need of removal of a large excess of unreacted reagent or use of N,N-dimethylformamide which is hard to recover.

The process of this invention will be described more particularly below by showing the examples thereof, which examples, however, are merely intended to be illustrative and not to be construed as limiting the scope of the invention.

Example 1

Into a mixed solution consisting of 156 parts by weight of sodium 1,2-naphthoquinonediazido-5-sulfonate monohydrate, 1,800 parts by weight of 1,2-dichloroethane and 11 parts by weight of pyridine (molar ratio of pyridine to sodium 1,2-naphthoquinonediazido-5-sulfonate: 0.25), 52 parts by weight (1.0 time the stoichiometric amount) of phosgene was blown over a period of 6 hours while maintaining the mixed solution at 10-15 °C under stirring, thereby effecting a sulfonyl chloride forming reaction. After the end of the reaction, the reaction mixture was washed three times with 1.5% hydrochloric acid at 10-15 °C to give a 1,2-dichloroethane solution of 1,2-naphthoquinonediazido-5-sulfonyl chloride.

The result of high performance liquid chromatographic analysis showed that the yield of the product based on the sodium 1,2-naphthoquinonediazido-5-sulfonate was 97%.

The 1,2-dichloroethane solution of 1,2-naphthoquinonediazido-5-sulfonyl chloride thus obtained was concentrated in vacuo at room temperature to 226 parts by weight. The concentrate was cooled to 5 °C and kept at 5 °C for an hour. The deposited crystals were separated by filtration and dried in vacuo at room temperature to give 128 parts by weight of 1,2-naphthoquinonediazido-5-sulfonylchloride (purity: 99.3%).

Example 2 and Comparative Example 1

The procedure of Example 1 was repeated except that the molar ratio of pyridine to sodium 1,2-naphthoquinonediazido-5-sulfonate was changed as shown in Table 1. The obtained results are shown in Table 1.

Table 1

| | Run No. | Molar ratio of pyridine to sodium 1,2-naphthoquinone-diazido-5-sulfonate | Yield of 1,2-naphthoquinone-diazido-5-sulfonyl chloride (%) | Purity of 1,2-naphthoquinone-diazido-5-sulfonyl chloride (%) |
|---|---|---|---|---|
| Example 2 | 1 | 0.35 | 97 | 99.6 |
| | 2 | 0.40 | 96 | 99.1 |
| | 3 | 0.90 | 96 | 99.3 |
| Comparative Example 1 | 1 | 0.10 | 84 | 99.3 |
| | 2 | 0.20 | 88 | 99.3 |

Comparative Example 2

Into a mixed solution consisting of 98 parts by weight of sodium 1,2-naphthoquinonediazido-5-sulfonate, 1,428 parts by weight of ethylene chloride and 111 parts by weight of N,N-dimethylformamide, 43 parts by weight (1.2 times the stoichiometric amount) of phosgene was blown over a period of 2 hours and 52

minutes while maintaining the mixed solution at 0-25 °C under stirring, thereby effecting a sulfonyl chloride forming reaction. After the end of the reaction, the reaction mixture was washed four times with 271 parts by weight of ice-cold water, then ethylene chloride was removed by distillation under reduced pressure at room temperature and the residue was dried in vacuo at room temperature. There was consequently obtained 90 parts by weight of 1,2-naphthoquinonediazido-5-sulfonyl chloride with a purity of 98.8%. The yield of the product based on sodium 1,2-naphthoquinonediazido-5-sulfonate was 92%.

**Claims**

1. A process for preparing 1,2-naphthoquinonediazido-5-sulfonyl chloride represented by the formula (2):

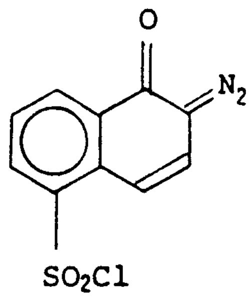

(2)

which comprises reacting 1,2-naphthoquinonediazido-5-sulfonic acid or a salt thereof represented by the formula (1):

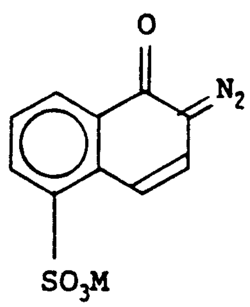

(1)

wherein M represents a hydrogen atom, a sodium or a potassium, with phosgene in the presence of pyridine.

2. The process according to Claim 1, wherein the compound of formula (1) is sodium 1,2-naphthoquinonediazido-5-sulfonate.

3. The process according to Claim 1 or 2, wherein the amount of phosgene used is 0.9-1.2 mole per mole of sulfonic group.

4. The process according to any one of Claims 1 to 3, wherein the amount of phosgene used is 0.95-1.15, preferably 1.0-1.1 mole per mole of sulfonic group.

5. The process according to any one of Claims 1 to 4, wherein the amount of pyridine used is 0.3-1, preferably 0.35-0.5 mole per mole of sulfonic group.

6. The process according to any one of Claims 1 to 5, wherein the reaction is carried out in a lower halogenated hydrocarbon.

7. The process according to Claim 6, wherein the lower halogenated hydrocarbon is methylene chloride, chloroform, carbon tetrachloride, 1,2-dichloroethane or perchloroethylene.

8. The process according to Claim 6, wherein the lower halogenated hydrocarbon is 1,2-dichloroethane.

9. The process according to any one of Claims 1 to 8, wherein the reaction is carried out at a temperature of 0 to 25 °C, preferably 5 to 20 °C, more perferably 10 to 15 °C.

10. The process according to any one of Claims 1 to 9, wherein the reaction is carried out under normal pressure.

**Patentansprüche**

1. Verfahren zur Herstellung von 1,2-Naphthochinon-diazido-5-sulfonylchlorid mit der Formel (2):

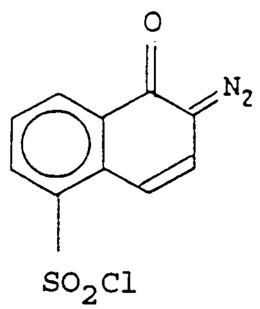

(2)

umfassend die Umsetzung von 1,2-Naphthochinon-diazido-5-sulfonsäure oder deren Salz mit der Formel (1):

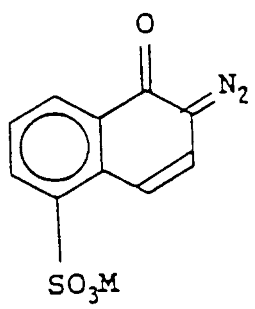

(1)

wobei M ein Wasserstoffatom, Natrium oder Kalium darstellt, mit Phosgen in Gegenwart von Pyridin.

2. Verfahren nach Anspruch 1, wobei die Verbindung der Formel (1) Natrium-1,2-naphthochinon-diazido-5-sulfonat ist.

3. Verfahren nach Anspruch 1 oder 2, wobei die verwendete Menge Phosgen 0,9 bis 1,2 Mol pro Mol Sulfonsäuregruppe ist.

4. Verfahren nach einem der Ansprüche 1 bis 3, wobei die verwendete Menge Phosgen 0,95 bis 1,15, vorzugsweise 1,0 bis 1,1 Mol pro Mol Sulfonsäuregruppe ist.

5. Verfahren nach einem der Ansprüche 1 bis 4, wobei die verwendete Menge Pyridin 0,3 bis 1, vorzugsweise 0,35 bis 0,5 Mol pro Mol Sulfonsäuregruppe ist.

6. Verfahren nach einem der Ansprüche 1 bis 5, wobei die Umsetzung in einem niederen halogenierten Kohlenwasserstoff durchgeführt wird.

7. Verfahren nach Anspruch 6, wobei der niedere halogenierte Kohlenwasserstoff Methylenchlorid, Chloroform, Tetrachlorkohlenstoff, 1,2-Dichlorethan oder Perchlorethylen ist.

8. Verfahren nach Anspruch 6, wobei der niederehalogenierte Kohlenwasserstoff 1,2-Dichlorethan ist.

**9.** Verfahren nach einem der Ansprüche 1 bis 8, wobei die Umsetzung bei einer Temperatur von 0 bis 25 °C, vorzugsweise 5 bis 20 °C, und stärker bevorzugt 10 bis 15 °C durchgeführt wird.

**10.** Verfahren nach einem der Ansprüche 1 bis 9, wobei die Umsetzung unter Normaldruck durchgeführt wird.

**Revendications**

**1.** Procédé de préparation de chlorure de 1,2-naphtoquinonediazido-5-sulfonyle représenté par la formule (2):

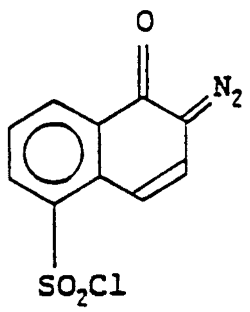

(2)

comprenant l'étape consistant à faire réagir de l'acide 1,2-naphtoquinone-diazido-5-sulfonique ou un sel de celui-ci, représenté par la formule (1):

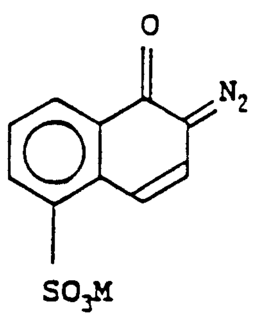

(1)

dans laquelle M représente un atome d'hydrogène, de sodium ou de potassium, avec du phosgène en présence de pyridine.

**2.** Procédé selon la revendication 1, dans lequel le composé de formule (1) est le 1,2-naphtoquinonediazido-5-sulfonate de sodium.

**3.** Procédé selon la revendication 1 ou 2, dans lequel la quantité de phosgène utilisée est de 0,9 à 1,2 mole par mole de groupement sulfonique.

**4.** Procédé selon l'une quelconque des revendications 1 à 3, dans lequel la quantité de phosgène utilisée est de 0,95 à 1,15, de préférence de 1,0 à 1,1 mole par mole de groupement sulfonique.

**5.** Procédé selon l'une quelconque des revendications 1 à 4, dans lequel la quantité de pyridine utilisée est de 0,3 à 1, de préférence de 0,35 à 0,5 mole par mole de groupement sulfonique.

**6.** Procédé selon l'une quelconque des revendications 1 à 5, dans lequel la réaction est conduite dans un hydrocarbure halogéné inférieur.

**7.** Procédé selon la revendication 6, dans lequel l'hydrocarbure halogéné inférieur est le chlorure de méthylène, le chloroforme, le tétrachlorure de carbone, le 1,2-dichloroéthane ou le perchloroétbylène.

8. Procédé selon la revendication 6, dans lequel l'hydrocarbure halogéné inférieur est le 1,2-dichloroéthane.

9. Procédé selon l'une quelconque des revendications 1 à 8, dans lequel la réaction est conduite à une température de 0 à 25 °C, de préférence de 5 à 20 °C et, avec une préférence particulière, de 10 à 15 °C.

10. Procédé selon l'une quelconque des revendications 1 à 9, dans lequel la réaction est conduite sous la pression normale.